# EUROPEAN PATENT SPECIFICATION

(11) **EP 1 644 505 B1**
(45) Date of publication and mention of the grant of the patent: **23.04.2008**
(21) Application number: 04737685.0
(22) Date of filing: 09.07.2004
(51) Int. Cl.: C12N 15/81, C12N 9/64, C07K 14/395, C07K 14/47, G01N 33/68

(54) **A YEAST MODEL FOR AMYLOIDOGENIC PROTEIN TOXICITY**
HEFEMODELL FÜR DIE TOXIZITÄT AMYLOIDOGENER PROTEINE
MODELE DE LEVURE POUR UNE TOXICITE DE PROTEINE AMYLOIDOGENIQUE

(30) Priority: 11.07.2003 EP 03447184
(43) Date of publication of application: 12.04.2006
(73) Proprietor: reMynd NV, 3000 Leuven (BE)
(72) Inventor: GRIFFIOEN, Gerard, B-3210 Linden-Lubbeek (BE); DUHAMEL, Hein, B-3010 Kessel-Io (BE); VAN DAMME, Nele, B-3010 Kessel-Io (BE); WINDERICKX, Joris, 3012 Wilsele (BE); WERA, Stefaan, B-3361 Bierbeek (BE)
(74) Representative: Bird, Ariane
(86) International application number: PCT/BE2004/000102
(87) International publication number: WO 2005/005640

(56) References cited:
- WO-A-02/065136
- HASHIMOTO M ET AL: "OXIDATIVE STRESS INDUCES AMYLOID-LIKE AGGREGATE FORMATION OF NACP/ALPHA-SYNUCLEIC IN VITRO" NEUROREPORT, RAPID COMMUNICATIONS OF OXFORD, OXFORD, GB, vol. 10, no. 4, 17 March 1999 (1999-03-17), pages 717-721, XP000891964 ISSN: 0959-4965
- KANDA S ET AL: "Enhanced vulnerability to oxidative stress by alpha-synuclein mutations and C-terminal truncation." NEUROSCIENCE. 2000, vol. 97, no. 2, 2000, pages 279-284, XP002306402 ISSN: 0306-4522
- KRUEGER R ET AL: "Involvement of alpha-synuclein in Parkinson's disease and other neurodegenerative disorders" JOURNAL OF NEURAL TRANSMISSION, SPRINGER VERLAG, VIENNA, AT, vol. 107, no. 1, 26 January 2000 (2000-01-26), pages 31-40, XP002201751 ISSN: 0300-9564
- STEECE-COLLIER KATHY ET AL: "Etiology of Parkinson's disease: Genetics and environment revisited." PROCEEDINGS OF THE NATIONAL ACADEMY OF SCIENCES OF THE UNITED STATES OF AMERICA, vol. 99, no. 22, 29 October 2002 (2002-10-29), pages 13972-13974, XP002306124 ISSN: 0027-8424
- OUTEIRO TIAGO FLEMING ET AL: "Yeast cells provide insight into alpha-synuclein biology and pathobiology." SCIENCE. 5 DEC 2003, vol. 302, no. 5651, 5 December 2003 (2003-12-05), pages 1772-1775, XP002306125 ISSN: 1095-9203
- WILLINGHAM STEPHEN ET AL: "Yeast genes that enhance the toxicity of a mutant huntingtin fragment or alpha-synuclein." SCIENCE (WASHINGTON D C), vol. 302, no. 5651, 5 December 2003 (2003-12-05), pages 1769-1772, XP002306126 ISSN: 0036-8075
- SCHERZER C R ET AL: "Yeast genetics targets lipids in Parkinson's disease" TRENDS IN GENETICS, ELSEVIER SCIENCE PUBLISHERS B.V. AMSTERDAM, NL, vol. 20, no. 7, July 2004 (2004-07), pages 273-277, XP004516781 ISSN: 0168-9525

### SEQUENCE LISTING

<110> reMYND N.V.
   Griffioen, Gerard
   Duhamel, Hein
   van Damme, Nele
   Winderickx, Joris
   Wera, Stefaan
<120> A YEAST MODEL FOR AMYLOIDOGENIC PROTEIN TOXICITY
<130> R2903-PCT
<150> EP 03447184.7
   <151> 2003-07-11
<160> 2
<170> PatentIn version 3.1
<210> 1
   <211> 39
   <212> DNA
   <213> Artificial Sequence
<220>
   <223> forward primer alpha-synuclein
<400> 1
   gatcagaatt cccatggatg tattcatgaa aggactttc 39
<210> 2
   <211> 41
   <212> DNA
   <213> Artificial Sequence
<220>
   <223> reverse primer alpha synuclein
<400> 2
   gatcaagatc tctcgagtta ggcttcaggt tcgtagtctt g 41

## Description

### Field of the invention

The present invention relates to a yeast model, and the use thereof for pharmaceutical screening, for modelling of neurodegenerative diseases and for cellular modelling of biochemistry of the amyloidogenic proteins involved therein.

### Background of the invention

Sporadic age-related neurodegenerative disorders are characterised by an abnormal high incidence of neuronal cell loss which, depending on the location in the brain afflicted, leads to memory loss and/or motoric and cognitive dysfunction. Aberrant aggregation of specific proteins (intra- or extra-cellular) is a common pathological hallmark of such diseases. For instance, brains from patients with Alzheimer's disease contain extra-neuronal plaques composed of beta-amyloid, and intracellular aggregates composed of another protein, called tau. Likewise, Parkinson's disease and Lewy body disease are characterised by intracellular precipitates and inclusions of alpha-synuclein (Taylor et al. (2002) Proc Natl Acad Sci USA 99,13972-4).

The underlying molecular mechanism responsible for development of these proteinaceous fibrils is largely unknown, but in many cases metal ions (among others triggers and risk factors) seem to play an important role.

A current hypothesis assumes that the protein aggregates or the aggregation process itself is neurotoxic. For instance, alpha-synuclein or beta-amyloid inclusions appear to promote neuronal dysfunction and oxidative stress. Other studies indicated that soluble oligomers of amyloidogenic proteins that are formed during the first steps of the aggregation process appear cytotoxic rather than the insoluble inclusions themselves. This prompted the suggestion that cytotoxicity exerted by various amyloidogenic proteins relates to a common mechanism. Protein aggregation is considered as a multistep complex presumably initiated by partial unfolding of amyloid-forming proteins which then further leads to the formation of oligomers and ultimately large aggregates. Risk factors (such as metal ions, ageing, mutations in the respective genes....) may promote unfolding and consequently fibril formation of amyloidogenic proteins.

Accumulation of unfolded proteins in the cell elicits a response aimed to remove these proteins. Indeed proteins in Alzheimer's disease (amyloid plaques), Parkinson's disease and Lewy bodies disease are ubiquitinated, presumably as part of a cellular defence mechanism to clear these denatured proteins via proteasome-mediated degradation. However, the protein inclusions are resistant to proteasomal protein degradation perhaps because the aggregates themselves inhibit the proteasomal machinery (Miller et al. (2003), Trends Pharmacol Sci 24,18-23; Snyder et al. (2003), J Biol Chem 278,11753-9). Interestingly in this context, many neurodegeneration-associated gene products are also a substrate for caspases, a group of proteases involved in the execution of apoptosis (Bredesen (1999) Neural notes (Promega) IV, 3-6)). At present the physiological relevance of caspase-mediated processing remains elusive.

Parkinson's disease is characterised by the selective loss of dopaminergic neurons in the *substantia nigra,* leading to dopaminergic deficits and motoric problems (Steece-Collier et al. (2002), Proc Natl Acad Sci USA 99:13972-13974). Inclusions of alpha-synuclein in substantia nigra neurons are a major pathological hallmark and play an essential role in the etiology of the disease. Several studies have shown that oxidative stress promotes the formation of toxic aggregates of alpha-synuclein. Iron, a redox active metal, also promotes aggregate formation through complexation with alpha-synuclein fibrils (Bush (2000), Curr Opin Chem Biol 4,184-91, Ostrera-Golts, cited above). In cells of the substantia nigra both risk factors are combined, since these neurons are enriched for iron and contain relatively high levels of radical-forming agents due to dopamine metabolism. This combination of risk factors might explain the selective degeneration of *substantia nigra* cells observed in Parkinson's disease (Steece-Collier, cited above).

The molecular mechanism of the cytotoxicity of alpha-synuclein fibrils remains elusive, alpha-synuclein fibrils might interfere with or inhibit proteasomal activity and thereby affect cell integrity. Alternatively, the aggregates themselves may generate oxidative stress, a process in which the complexed iron could play a pivotal role (Turnbull *et al*., cited above). The recent observation that the MPTP (1-methyl-4-phenyl-1,2,3,6-tetrahydropyridine) induced loss of dopaminergic neurons in wild type mice (MPTP elicits an acute intracellular oxidative stress) is suppressed in alpha-synuclein null mice dramatically demonstrates the key role of alpha-synuclein in the etiology of Parkinson's disease (Dauer et al. (2002), Proc Natl Acad Sci USA 99,14524-14529).

Different types of animal models have been developed for the study of neurodegenerative diseases, based on either transgenic or toxin-induced approaches. An animal model for Parkinson's disease has been developed in mice and rats by injecting viral vectors encoding wild type or clinical mutants of alpha-synuclein into the substantia nigra of these animals (Kirik et al. (2003) PNAS 100, 2884-2889). The resulting overexpression of alpha synuclein results in time-dependent neurological changes reminiscent of Lewy pathology and can be used for the screening of potential therapeutics. Though these models are in most cases capable of replicating most of its clinical and pathological hallmarks of the disease, the inherent time and infrastructure constraints make them less suitable for high-throughput screening. The gene encoding alpha-synuclein has been transferred into the *Drosophila melanogaster* genome, whereby transgenic flies also recapitulate the essential features of Parkinson's disease and has been suggested as a screening assay for drugs (Pendleton et al. (2002) J Pharm Exp Therapeut 300, 91-96).

Yeast has proven to be a good vehicle for the expression of human proteins, based on the strong similarity of yeast and human cells on the molecular level. A lot of information has been obtained in this way on the interaction of proteins and DNA in cellular mechanisms involved in normal processes and/or human diseases. Wild-type and mutant alpha-synuclein have been expressed in yeast with the object of analysing the regulation of synuclein misfolding (http://www.mcmp.purdue.edu/faculty/rochet.php). Expression of different domains of wild-type alpha-synuclein in *E coli* led to the identification of a protease sensitive C-terminal truncation. The use of a two-hybrid screen in yeast has made it possible to identify synphilin-1 as protein interaction partner of alpha-synuclein (Neystat et al. (2002) Neurosci Lett 325, 119-123).

WO 02/065136 describes the use of yeast as a model for screening compounds or drug targets with therapeutic value for diseases associated with protein aggregation. This involves the expression of amyloidogenic peptides in yeast which is contacted with an agent that induces toxicity in the yeast cell. Examples of such toxicity inducing agents are carbon sources, metal ions etc..

### Summary of the invention

The present application describes a yeast model recapitulating the cytotoxic effects of amyloidogenic proteins. This model consists of yeast cells in which an amyloidogenic protein is heterologously expressed and whereby fibril formation and/or aggregation of such a protein results in a phenotypic read-out, including, but not limited to cell survival, proliferation, reporter gene activity or enzymatic activity of a reporter protein.

The present invention provides a yeast model for neurodegenerative diseases in which the toxic effects of amyloidogenic proteins are more pronounced, which is advantageous both for the analysis of the mechanism of neurodegenerative diseases and the screening of potential (precursors of) therapeutic agents or drug targets for use in the treatment of amyloidogenic neuropathies. More particularly, the present invention provides an engineered yeast cell having the features as described in the appended claims.

The present invention is based on the observation that expression of amyloidogenic proteins in wild type yeast results in amyloid fibril formation and/or aggregation of these proteins providing a phenotypic read-out. More particularly it was found that expression of such proteins in wild type yeast can be toxic. A dominant negative effect of the amyloidogenic proteins is evidenced from the observation that proliferation of yeast cells expressing the gene encoding these proteins or mutant derivatives thereof is reduced relative to control strains especially when metal ions, such as zinc ions (Zn2+) or iron ions (Fe3+) are supplemented to the growth medium.

This metal ion-dependent toxicity of amyloidogenic proteins in yeast resembles the degeneration seen in the brains of patients of neurodegenerative diseases. More particularly, the metal ion-dependent toxicity of alpha-synuclein mimics the degeneration seen in the substantia nigra cells in Parkinson's disease brains since iron-stimulated formation of deleterious alpha-synuclein fibrils appears to be mainly responsible for the loss of dopaminergic neurons. Thus, the present invention relates to the use of an engineered yeast cell as characterized in claim 1 as a yeast model for amyloidoses, such as Parkinson's disease and Lewy body disease, wherein the toxic expression of amyloidogenic proteins mimics the neurotoxic effects in diseased brain.

This application further describes expression of genes or minigenes encoding amyloidogenic proteins in a strain lacking a functional gene responsive to oxidative stress. More particularly, a yeast strain which as a result of a deletion mutation lacks a functional yeast caspase gene (*yca1*), was found to display an increased sensitivity to the expression of amyloidogenic proteins. Expression of such proteins in a mutant strain such as *yca1,* leads to a more severe inhibition of growth compared to wild type yeast strains that express a functional caspase gene. More particularly it was found that, even in the absence of metal ions in the growth medium, proliferation of a *yca1* strain expressing wild type human amyloidogenic proteins, such as wild-type human alpha-synuclein, is strongly reduced. This demonstrates that yeast genes responsive to oxidative stress, such as the gene encoding caspase, play a role in the molecular mechanism that renders amyloidogenic proteins toxic in yeast cells.

Thus according to an aspect of the invention, yeast strains lacking a functional gene responsive to oxidative stress which is a caspase Yca1 gene, expressing one or more amyloidogenic proteins or mutant derivatives thereof are used as a model for the cytotoxicity of these amyloidogenic proteins in mammals. More particularly yeast strains lacking a functional caspase Yca1 gene, such as the mutant strains *yca1* are provided, which are particularly sensitive to the expression of amyloidogenic proteins.

The more pronounced cytotoxic character of amyloidogenic proteins such as alpha-synuclein in *yca1* yeast cells compared to wild type cells highly facilitates high-throughput screens aimed to identify chemical compounds or human cDNA's that reverses or suppresses presumed cytotoxic effects of these proteins. Firstly, cytotoxicity can be detected with relatively low levels of fibril promoting reagents (such as metal ions) or possibly even without these agents. This results in a lower frequency of false positives in high-throughput screens since hits that neutralise the effects of the fibril promoting reagents but do not suppress toxicity of amyloidogenic proteins as such are not identified. Secondly, the increased cytotoxicity of alpha-synuclein results in a larger difference in growth between alpha-synuclein producing strains and control strains (i.e. the growth read-out is more sensitive). The result of this is a more stringent assay for the identification of compounds (including cDNA's) that modulate the toxic effects of amyloidogenic proteins, reducing the number of false positives. On the other hand, as the assay is more sensitive, compounds that have a more limited modulating effect on amyloidogenic protein toxicity, are more likely to be identified, resulting in less false negatives or more true positive hits. Accordingly, the invention relates to the use of the yeast cell as characterized in claims 1 to 3 for screening compounds capable of affecting the toxicity of amyloidogenic proteins in yeast.

Thus the present invention relates to a method for identifying compounds that modulate the toxic effects of amyloidogenic proteins, said method comprising the steps of:
a) providing an engineered yeast strain, comprising a transgene encoding one or more amyloidogenic proteins or mutant(s) thereof, wherein said yeast strain is characterized in that it lacks a functional Yca1 gene. Herein the one or more amyloidogenic proteins can be encoded by a minigene.
b) contacting the yeast strain obtained in step (a) with said compound, and
c) determining the phenotypic effect of said compound on said yeast.

Specific embodiments of this method are described in claims 8 and 9.

### Detailed description of the invention

The present application describes a yeast model recapitulating the neurotoxic effects of amyloidogenic proteins. This model consists of yeast cells in which one or more amyloidogenic proteins are heterologously expressed and whereby this expression has a phenotypic effect on the yeast cells, so that the influence of different compounds or groups of compounds or other molecules on the expression or the activity of the amyloidogenic proteins on this phenotypic effect can be assayed. In order to obtain such a phenotypic read-out the amyloidogenic protein is expressed in a specific genetic background, consisting of yeast strains carrying mutations in one or more functional yeast genes and/or expressing one or more heterologous genes. More particularly, mutations in genes responsive to oxidative stress were found to significantly sensitise the yeast to expression of amyloidogenic proteins. The resulting strain is grown on classic media or on specifically developed media, including media such as, but not limited to, media inducing oxidative stress through the presence of metal ions, paraquat, rotenone or other oxidative stressors.

Thus, the present application describes mutant yeast cells that display increased toxicity towards amyloidogenic proteins involved in neurodegeneration.

The term **'model'** as used herein relates to a system which mimics at least one aspect of the process of interest. More particularly, in the context of the present invention yeast cells are provided in which the expression of amyloidogenic proteins results in a phenotype which is similar to that observed in certain cells of the brain in human and animal amyloidosis disorders. More particularly, cytotoxicity profile of amyloidogenic proteins in the yeast cells of the invention is correlated with the neurodegeneration caused by amyloidogenic proteins in these diseases. Applications of this model include, but are not limited to, the investigation of the factors involved the cytotoxicity of amyloidogenic diseases, the investigation into the biochemistry of the amyloidogenic proteins and the testing of compounds for their ability to influence the observed phenotypic effect of amyloidogenic proteins (e.g. pharmaceutical screening).

The term **"yeast cell"** herein is used to mention unicellular fungi of the phylum Ascomycota that reproduce by fission or budding and are capable of fermenting carbohydrates into alcohol and carbon dioxide. Preferably said yeast cells are *Saccharomycetales* or *Schizossaccharomycetales,* such as *Saccharomyces pombe, Saccharomyces cerevisae, Candida* or *Pichia.* Most preferably yeast cells of the species *Saccharomyces cerevisae* are used for manipulation in accordance with the present invention. A genetically stable yeast cell is also referred to as a yeast strain.

In the context of the present invention, yeast strains **"lacking one or more functional genes"** are yeast strains in which one or more genes cannot be expressed (no gene product) or which express an aberrant gene product, i.e. a product which is not the natural gene product. Preferably, according to the present invention, such an aberrant gene product, is a gene product which has lost its original function (as present in the wild-type strain). Alternatively such aberrant gene product has a reduced function (less than 50%, less than 75%, or even less than 90%) when compared to the wild type function (enzymatic activity, binding affinity etc.) Yeast cells or strains lacking one or more functional genes can be the result of different factors, such as, but not limited to one or more mutations in the gene itself or in a gene encoding a factor (protein, RNA, dsRNA) which is capable of modifying the expression of said gene; a truncation (caused e.g. by aberrant expression of an enzyme capable of degrading said protein); the complete absence of a gene; the insertion of a transposon; expression of a transgene (resulting in missense, antisense, sense expression) which modifies the expression or the activity of the natural gene product (see below).

A **"mutant yeast strain"** refers to a strain which, as a result of either natural processes or mutagenesis techniques including but not limited to site-directed mutagenesis, contains one or more mutations (deletions, insertions and/or subsitutions) in its genome as compared to the wild-type strain (used for its taxonomic classification). Preferred embodiments of such mutant strains according to the present invention are deletion (yeast) strains, more particularly, deletion strains comprising one or more deletions in one or more genes responsive to oxidative stress, resulting in the strain lacking one or more functional genes responsive to oxidative stress. A functional gene, as used herein refers to a gene which is capable of expressing its natural gene product. Preferred embodiments of such deletion strains are the *yca1* strains, bearing one or more deletions for a yeast homologue of mammalian caspase, such as *YCA1* (encoded by ORF: YOR197W) and sod strains, bearing a deletion for a yeast homologue of superoxide dismutase such as SOD1 (*sod1* strain) (encoded by ORF: YJR104C) or SOD2 (*sod2* strain)(encoded by ORF: YHR008C). Other examples of genes which are responsive to oxidative stress are reviewed in Godon et al. J. Biol. Chem. 273 (1998) 22480-22489. In this comprehensive study many genes have been identified that respond to H₂O₂.

A preferred example of a mutant *yca1* strain is the mutant *yca1* strain described by Giaever et al. (2002) Nature 418, 387-391). Alternatively, genomic deletions of specific genes can be made in suitable yeast strains by methods known in the art, such as, but not limited to, PCR product-directed gene disruption.

The present application relates to yeast cells which are defective for one or more genes which are responsive to oxidative stress. Genes responsive to oxidative stress are genes which are either up- or down-regulated at the transcriptional level by a factor of at least 2 as a result of oxidative stress (Gash et al. (2000), Mol Biol Cell 11. 4241-4257) and/or of which expression of the gene product is influenced by oxidative stress and/or of which the activity of the gene product (e.g. enzymatic activity) is influenced by oxidative stress (such as Yca1). Typical compounds which provoke oxidative stress are for example hydrogen peroxide, diamide, menadione, redox-active metal ions (Fe^{2+/3+}, Cu^{2+/3+})].

According to a particular aspect of the present invention yeast cells are manipulated to incorporate DNA sequences, more particularly DNA sequences encoding amyloidogenic proteins or parts thereof. Yeast cells do not normally express human amyloidogenic proteins but are capable of expressing such proteins by introduction of a DNA sequence encoding them under the control of appropriate regulatory DNA sequences. Alternatively, endogenous DNA sequences can be introduced under control of appropriate regulatory sequences to obtain overexpression of endogenous genes. The resulting DNA sequence is considered a **"recombinant"** DNA sequence or a **"transgene".** The yeast strain comprising the recombinant DNA stably integrated in its genome can be referred to as a recombinant yeast strain.

Thus, the term **"transgene"** refers to any piece of nucleic acid (DNA or RNA) which has been inserted into a cell (e.g. by transformation), and which may or may not be incorporated into the genome. Preferably, the transgene becomes part of the genome of the organism (i.e. either stably integrated or as a stable extrachromosomal element). Such a transgene includes genes which are partly or entirely heterologous (i.e. foreign) as well as genes homologous to endogenous genes of the organism. Including within this definition is a transgene created by providing an RNA sequence which is reverse transcribed into DNA and then incorporated into the genome, or an antisense agent or molecule. Such a transgene may or may not encode a gene product and/or may modify the expression of an endogenous gene product. The nucleic acid sequence may be introduced by any suitable transformation technique including electroporation, chemical transformation methods such as the Litium Acetate method or other techniques known to those skilled in the art.

The term **"engineered yeast"** is used herein to mention yeast cells or strains, having a transgene or non-endogenous (i.e. heterologous) nucleic acid sequence present as a extrachromosomal element or stably integrated into its germ line DNA (i.e. in the genomic DNA).

An **"amyloidogenic protein"** as used herein refers to a protein that is involved in the deposition of insoluble amyloid fibres (which are also specifically referred to as amyloidoses). Examples of such amyloidogenic proteins include, but are not limited to huntingtin, alpha-synuclein, tau and beta-amyloid ataxin, SOD1, transthyretin, serum amyloid A, islet amyloid polypeptide, cystatin-C and lysozyme. More particularly, the amyloidogenic proteins contemplated in the context of the present invention are amyloidogenic proteins involved in neurodegenerative diseases, such as alpha-synuclein, tau huntingtin, and beta-amyloid.

The term **"alpha-synuclein"** refers to the pre-synaptic protein which has been identified as the main component in Lewy bodies in both inherited and sporadic Parkinsons Disease.

The term **"alpha-synuclein gene"** refers to a nucleic acid sequence encoding the alpha-synuclein protein, an isoform or functional homologue thereof, such as the human NACP/alpha-synuclein gene or an allelic variant or minigene thereof.

The terms **"tau", "protein tau"** or **"tau-protein"** refer to a specific (poly)peptide or protein associated with the assembly, stability, or enhancement of the polymerisation of microtubules. Tau protein exists in up to 6 different isoforms in human adult brain, while only 1 isoform is expressed in fetal brain, but all are generated from a single gene on human chromosome 17 by alternative mRNA splicing. Tau exists in several phosphorlylated forms as reviewed for example in Geschwind (2003) Neuron. 40, 457-460.

The term **"tau gene"** as used herein refers to a nucleic acid sequence encoding the tau protein, an isoform or functional homologue thereof, such as the gene encoding htau40, or an allelic variant or minigene thereof. A particular embodiment refers to mutated versions mimicking phosphorylated or unphosphorylated versions of tau.

As used herein, the term **"minigene"** refers to a heterologous gene construct wherein one or more nonessential segments of a gene are deleted with respect to the naturally-occurring gene. Typically, deleted segments are intronic sequences of at least about 100 basepairs to several kilobases, and may span up to several tens of kilobases or more. Isolation and manipulation of large (i.e., greater than about 50 kilobases) targeting constructs is frequently difficult and may reduce the efficiency of transferring the targeting construct into a host cell. Thus, it is frequently desirable to reduce the size of a targeting construct by deleting one or more nonessential portions of the gene. Typically, intronic sequences that do not encompass essential regulatory elements may be deleted. Frequently, if convenient restriction sites bound a nonessential intronic sequence of a cloned gene sequence, a deletion of the intronic sequence may be produced by: (1) digesting the cloned DNA with the appropriate restriction enzymes, (2) separating the restriction fragments (e.g., by electrophoresis), (3) isolating the restriction fragments encompassing the essential exons and regulatory elements, and (4) ligating the isolated restriction fragments to form a minigene wherein the exons are in the same linear order as is present in the germline copy of the naturally-occurring gene. Alternate methods for producing a minigene will be apparent to those of skill in the art (e.g., ligation of partial genomic clones, which encompass essential exons but which lack portions of intronic sequence). Most typically, the gene segments comprising a minigene will be arranged in the same linear order as is present in the germline gene, however, this will not always be the case. Some desired regulatory elements (e.g., enhancers, silencers) may be relatively position-insensitive, so that the regulatory element will function correctly even if positioned differently in a minigene than in the corresponding germline gene. For example, an enhancer may be located at a different distance from a promoter, in a different orientation, and/or in a different linear order. For example, an enhancer that is located 3' to a promoter in germline configuration might be located 5' to the promoter in a minigene. Similarly, some genes may have exons, which are alternatively spliced, at the RNA level, and thus a minigene may have fewer exons and/or exons in a different linear order than the corresponding germline gene and still encode a functional gene product. A cDNA encoding a gene product may also be used to construct a minigene. However, since it is often desirable that the heterologous minigene be expressed similarly to the cognate naturally-occurring non-human gene, transcription of a cDNA minigene typically is driven by a linked gene promoter and enhancer from the naturally-occurring gene. Frequently, such minigene may comprise a transcriptional regulatory sequence (e.g., promoter and/or enhancer) that confers neuron-specific or CNS-specific transcription of the minigene alpha-synuclein encoding sequences. In a particular embodiment of a minigene, all intronic sequences have been removed.

A **"mutant gene"** refers to a gene of which the sequence has been altered with respect to the wild-type gene (N terminal deletions, C terminal deletions, internal deletions, substitutions (either silent or changing the encoded amino acid) at one or more positions, and combinations thereof). This term is mainly used in the present invention in the context of genes encoding human amyloidogenic proteins. Preferably, according to the present invention mutant genes refer to genes of which the coding sequence has been altered with respect to the wild-type human gene present in non-pathological conditions in human cells. Such mutant genes can be obtained by different *in vitro* (or *in vivo)* methods including genetic engineering or random or site-directed mutagenesis or can occur in nature, such as clinical mutants. The mutant proteins referred to herein are the proteins encoded by the respective mutant genes. The present invention relates, inter alia, to the expression in yeast of mutant genes of amyloidogenic proteins, such as mutant alpha-synuclein genes, which are preferably clinical mutations of the alpha-synuclein gene, such as, but not limited to A53T and A30P. In a particular embodiments mutations are introduced at positions, which mimic or prevent post-translational modifications such as phosphorylation.

A **"supplement"** as used herein is a compound that is added to classical yeast growth media. According to the present invention, supplements are provided that affect growth of yeast cells expressing amyloidogenic proteins such as those described in the present invention. These supplements include, but are not limited to, metal salts, such as FeCl₃, ZnCl₂ and paraquat.

A **"phenotypic effect"** as used herein relates to an effect on the yeast cells. Preferably this monitoring can be carried out directly, more particularly without molecular analysis. Such effects include but are not limited to effects on cell survival (cytotoxic effect) and cell proliferation (growth inhibition) or morphology but also effects that can be measured by reporter gene activity or enzymatic activity of a reporter protein. According to the present invention expression in yeast cells, of amyloidogenic proteins in wild-type or deletion strains, has a particular phenotypic effect on these cells. According to a particular aspect of the invention the phenotypic effect observed in the yeast strains is an effect on growth, more particularly a toxic effect, resulting in decreased cell proliferation and cell death. Alternatively, monitoring of the phenotypic effect can be performed indirectly i.e. through a parameter related to increased growth. Examples of parameters related to increased growth are consumption of components in the medium (amino acids, carbon source), level of metabolites, or reporter constructs (for instance with metabolic enzymes). A reporter gene construct (e.g. a nutrient marker or an enzyme-encoding gene) as used herein relates to promoter sequence of a gene of interest, i.e. of which expression is to be monitored, which is linked to a coding sequence of which the expression is easily detectable. Examples of such coding sequences are sequences encoding an enzyme (LacZ, X-gal digested will turn blue), or nutrient marker (such as *HIS,* or *LEU2* encoding the biosynthetic enzymes necessary for the production of the amino acids histidine and leucine, respectively). A reporter gene can be a transgene (eg LacZ) behind a suitable promoter. The reporter gene can also be a endogenous yeast gene which is up- or down-regulated. Suitable techniques for detecting reporter protein activity in cells are known in the art.

The term **"compound"** is used herein to denote a chemical structure, a mixture of chemical structures, a peptide, a biological substance such as a macromolecule, or an extract made from biological materials such as bacteria, plants, fungi, or animal (particularly mammalian) cells or tissues. According to one aspect of the present invention, methods are provided for identifying compounds capable of influencing, modifying (increasing or decreasing) the phenotypic effect of the expression of amyloidogenic proteins in yeast. The influence of a compound on the phenotypic effect observed in yeast cells can be direct, by interacting with the expressed amyloidogenic protein, or can be indirect, by affecting the expression of the amyloidogenic protein in yeast, or by affecting inhibitors or enzymes which themselves influence the phenotypic effect of the amyloidogenic proteins. Alternatively, the effect of these compounds may be by interacting with proteins or enzymes which mediate the phenotypic effect of the amyloidogenic protein. Such compounds include but are not limited to proteins or polypeptides (such as heat shock proteins, receptors, enzymes, ligands, nuclear or cytoplasmic proteins, chaperones, hormones, antigens, growth factors, regulatory proteins, structural proteins) nucleic acids (RNAs, ribozymes, DNAs or cDNAs) or chemical compounds. The screening of compounds from natural or synthetic libraries is also envisaged. cDNA targets encoding an RNA, protein or polypeptide capable of influencing, modifying the phenotypic effect of the expression of amyloidogenic proteins in yeast can be identified in the context of the present invention by transformation of a yeast strain with a cDNA library, and screening of the phenotype of the resultant yeast colonies.

A **'screening method'** as used herein refers to a method for identifying specific compounds. More particularly, the screening method of the present invention allows the identification of compounds based on their ability to modulate the toxic effect in yeast of the expression of amyloidogenic proteins. Screening kits referred to in the present invention can comprise one or more components of the invention such as, but not limited to, the mutant yeast strain of the invention, the mutant yeast strain engineered to express one or more amyloidogenic proteins, one or more supplements and optionally reagents for determining said phenotypic effects, e.g. reagents for detecting the viability of the cells or reagents for detecting the activity of an enzymatic reporter protein.

According to the present invention, screening may be carried out as follows: Yeast cells of the present invention expressing the amyloidogenic protein(s) display a particular phenotypic effect. The yeast cells are contacted with a medium comprising the compound for a specified time under specified conditions, and the phenotypic effect of the compound on the cells is evaluated either by direct measurement of cell viability (e.g. by measuring growth on solid medium, or by OD measurement) or by detecting the activity of a reporter protein.

Thus, the present application relates to a method for assessing the activity of candidate substances as modulators, more particularly inhibitors, of the cytotoxicity of the expression of one or more amyloidogenic proteins, which method comprises the steps of
a) providing a yeast strain lacking one or more functional genes responsive to oxidative stress
b) contacting the yeast strain with a medium comprising one or more candidate compounds, as well as with a control medium
c) comparing the growth of the yeast strain in the presence or absence of the one or more candidate compounds
d) determining the activity of the one or more candidate compounds, whereby a change in growth or a parameter related to a change in growth compared to control is an indication of modulation of cytotoxicity by the one or more candidate compounds; and optionally
e) selecting a compound which modulates, preferably inhibits, the cytotoxicity of the amyloidogenic protein(s) in the yeast strain.

More particularly a method for identifying a compound which influences the toxic effect of amyloidogenic proteins in yeast is claimed, the method comprising the steps of:
a) providing an engineered yeast strain, comprising a transgene or a minigene encoding one or more amyloidogenic proteins or mutant(s) thereof, wherein the yeast strain is characterized in that it lacks a functional Yca1 gene,
b) contacting the yeast strain obtained in step (a) with the compound, and
c) determining the phenotypic effect of the compound on the yeast strain.

According to a specific embodiment of the present invention, the effect of a compound on the toxicity of amyloidogenic proteins on the cells can be determined by contacting the yeast cells with a medium comprising a compound at 30°C for a period of 24-48 hours. Serial dilutions of liquid precultures can be spotted on solid media with or without the compound(s) followed by a 24-48h incubation at 30°C, and determining of cell culture density at OD₆₀₀. Increased growth rate compared to control is indicative of a compound (or combination thereof) with a potential of reducing toxicity. Similarly, reduced growth is indicative of one or more compound(s) which increase toxicity. Optionally, supplements are also added to the medium, which may also modulate the toxic effect of expression of amyloidogenic proteins. Yeast can also be put under additional stress by physical parameters such as elevated or reduced temperatures or elevated pressure. Such experiments can be carried out on a large scale, whereby the screening of large libraries of compounds is envisaged. According to the present invention, a more sensitive screening method is presented, allowing for a selective identification of compounds affecting toxicity of amyloidogenic proteins per se (and not compounds that neutralise the effects of fibril promoting reagents such as metal ions, but do not suppress toxicity of amyloidogenic proteins). The higher sensitivity results not only in a lower detection level, resulting in less false-negatives but also in a more stringent assay, reducing the number of false positives. The increase in sensitivity to amyloidogenic proteins compared to a yeast strain not lacking a functional gene responsive to oxidative stress is envisaged between 2x and 50x, optionally at least 5x. Thus, the present yeast model provides a sensitive high-throughput cost-effective screening method for the identification of compounds useful in the treatment, prevention and cure of diseases due to amyloid protein misfolding and/or aggregation.

The present application further envisages compounds identified by the screening method of the present invention said compounds being used for the treatment, prevention and cure of diseases due to amyloid protein misfolding and/or aggregation.

The yeast cells of the present invention may serve as models for diseases involving amyloidogenic proteins (also referred to as **'protein-aggregation diseases').** A preferred embodiment of the present invention relates to yeast cells as models for neurodegenerative disorders in which amyloidogenic proteins are involved; these conditions include but are not limited to Parkinson's disease, Alzheimer's, Huntington's disease, familial amyloid neuropathy, and transmissible spongiform encephalopathies. Accordingly, the yeast cells of the present invention may be used for the study of such conditions and screening of therapeutic agents for such conditions.

A **"fragment"** of a DNA molecule or protein sequence as used herein refers to a truncated sequence of the original (nucleic acid or amino acid) sequence referred to, which can vary in length but of which the minimum size is sufficient to ensure the (encoded) protein to be biologically active, the maximum size not being critical.

**"sequence identity"** of two sequences as used herein relates to the number of positions with identical nucleotides or amino acids divided by the number of nucleotides or amino acids in the shorter of the sequences, when the two sequences are aligned. Preferably said sequence identity is higher than 70%-80%, preferably 81-85%, more preferably 86-90%, especially preferably 91-95%, most preferably 96-100%, more specifically is 100%.

As used herein **"comprising"** is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA sequence which is functionally or structurally defined, may comprise additional DNA sequences, etc.

Unless otherwise stated, all recombinant DNA techniques are carried out according to standard protocols as described in Sambrook et al. (1989) Molecular Cloning: A Laboratory Manual, Second Edition, Cold Spring Harbour Laboratory Press, NY and in Volumes 1 and 2 of Ausubel et al. (1994) Current Protocols in Molecular Biology, Current Protocols, USA. Standard materials and methods relating to yeast genetics are described in Methods in yeast genetics: a Laboratory Course Manual. Cold Spring Harbour Laboratory Press, NY.

### Brief description of the Figures

The above detailed description, given by way of example, but not intended to limit the invention to specific embodiments described therein, may be understood in conjunction with the accompanying Figures, in which:

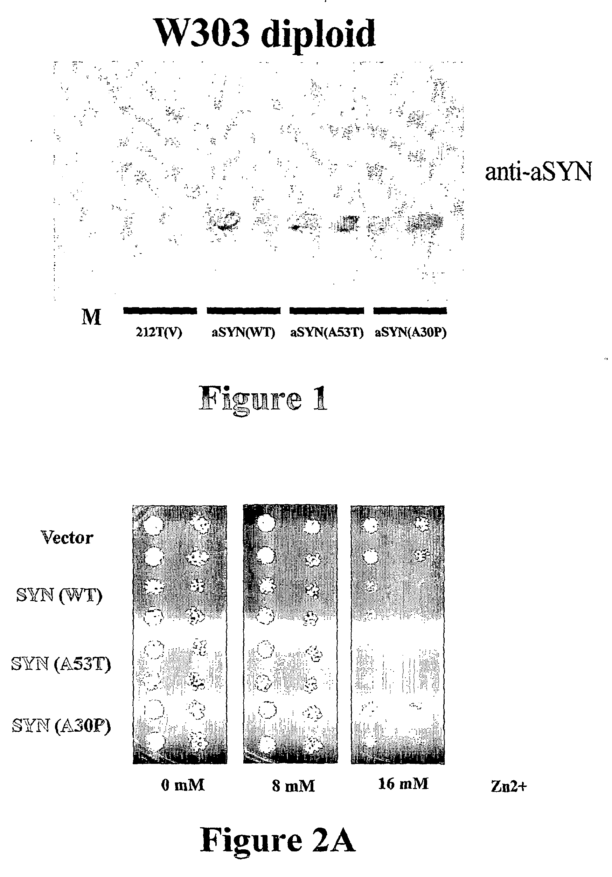

- Figure 1:: Western analysis of yeast cells expressing the human alpha-synuclein gene and mutant versions thereof. 212T(V) refers to diploid strain (W303-1A genetic background) transformed with a control vector which does not contain the gene encoding alpha-synuclein or transformed with plasmids 212T-aSYN(WT), 212T-aSYN(A53T) and 212T-aSYN(A30P) bearing cDNAs of human alpha-synuclein cDNA, or the clinical mutants alpha-synuclein (A53T) and alpha-synuclein (A30P), respectively.

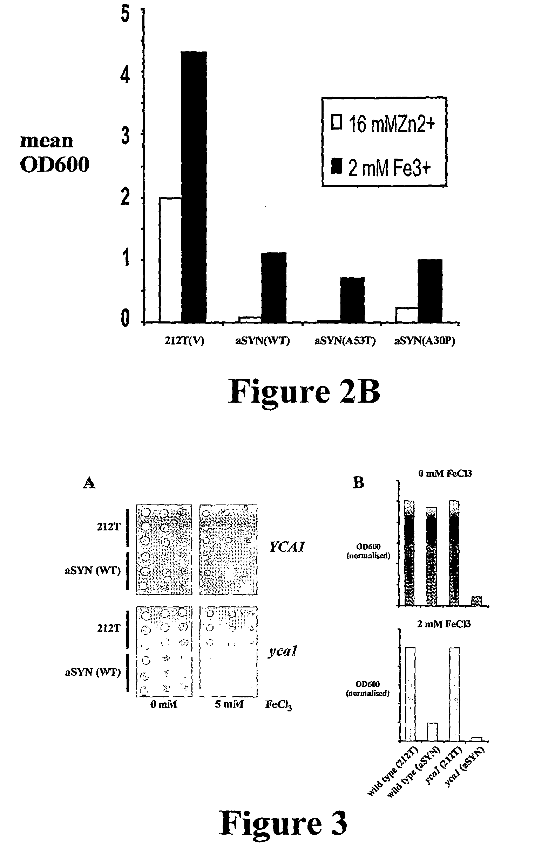

- Figure 2:: Growth characteristics of yeast cells expressing the human alpha-synuclein gene and mutant versions thereof on solid (A) or liquid (B) media containing FeCl₃. The same strains are used as described in the Legend to Figure 1. In (B) open and black bars indicate growth in the presence of 16 mM ZnSO₄ and 2 mM FeCl₃, respectively.
- Figure 3:: Growth characteristics of wild type strain *YCA1* (BY4147 background) and strain *yca1,* an isogenic strain deleted for the gene *YCA1* expressing human alpha-synuclein gene, according to an embodiment of the present invention, grown on solid (A) or liquid (B) media containing FeCl₃. 212T and aSYN refer to strains transformed with a control vector and plasmid 212T-aSYN containing alpha-synuclein cDNA, respectively.

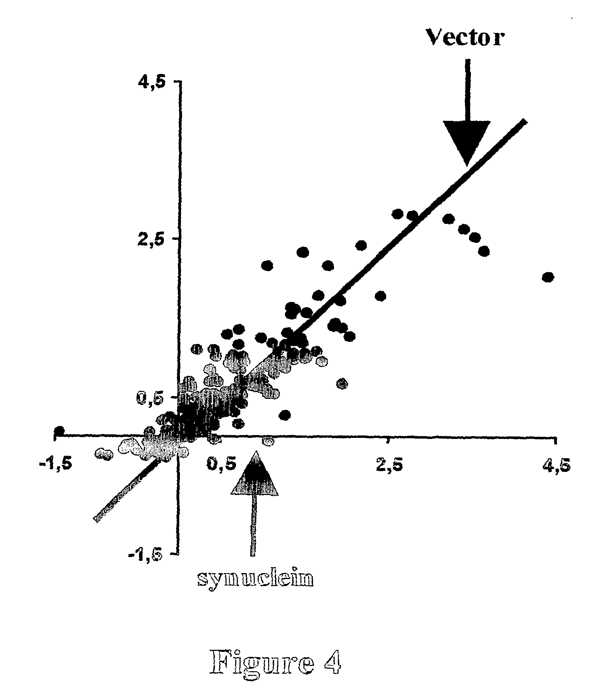

- Figure 4: :Characteristics of a yeast-based α-synuclein-opathy model in a HTS setting. The data are depicted as scatter graph in which the X and Y-axes represent the normalised values of each compound obtained from screen 1 and 2, respectively. The arrow "synuclein" and "vector" refer to the normalised growth value of the negative control (value = 1) and positive control (value = 3,5), respectively.

In the description and examples, reference is made to the following sequences:
- SEQ ID No 1:: forward primer alphaSYN1-F
- SEQ ID No 2:: reverse primer alphaSYN2-R

### Examples

### GENERAL METHODOLOGIES USED IN THIS INVENTION

**Yeast media and culture conditions:** Yeast cells were cultured at 30°C in standard yeast media as described elsewhere (Methods in Yeast Genetics, cited above). Synthetic complete (SC) medium supplemented with adenine and amino acids but lacking uracil was used to select for the alpha-synuclein expression plasmids as described in this invention. For metal ion toxicity studies in liquid culture 8 to 32 mM ZnSO₄ (MERCK; cat. No. 1.08883) or 1 to 4 mM FeCl₃ (Sigma-Aldrich; Cat. No. 20,792-6) were added to the medium. FeCl₃-containing medium was filtered prior to use. Growth tests were started at a cell density corresponding to an OD₆₀₀ of 0.1. Growth tests were performed at 30°C in microtiter plates in 150 µl volumes with incubation times between 24 h and 48 h. Toxicity studies on plates were done by spotting serial dilutions of liquid precultures on solid medium containing 5 mM FeCl₃, followed by a 48h incubation at 30°C.

**cDNA expression constructs:** cDNA encoding wild type human alpha-synuclein and mutant derivatives encoding A53T and A30P substitutions were constitutively expressed in yeast cells using the triose phosphate isomerase (*TPI1*) promoter and alcohol dehydrogenase (*ADH1*) terminator. To this end, a DNA fragment containing the *ADH1* terminator was cloned in the Xhol site of pYX212 (R&D systems Europe Ltd., UK) resulting in plasmid 212T. Subsequently, PCR fragments were generated encoding human alpha-synuclein and mutant derivatives with primers: alphaSYN1-F 5'-GAT CAG AAT TCC CAT GGA TGT ATT CAT GAA AGG ACT TTC-3' (SEQ ID NO: 1) and alphaSYN2-R 5'-GAT CAA GAT CTCT CGA GTT AGG CTT CAG GTT CGT AGT CTT G-3' (SEQ ID NO: 2) using wild type alpha-synuclein, alpha-synuclein(A53T) and alpha-synuclein(A30P) cDNA as template. These PCR fragments were subsequently cloned (Ncol/Xhol) in vector 212T resulting in plasmids 212T-aSYN(WT), 212T-aSYN(A53T) and 212T-aSYN(A30P). These alpha-synuclein expression plasmids were transformed to appropriate yeast strains.

**Yeast deletion strains:** A large number of yeast deletion mutants have been described by Giaever et al. (2002) Nature 418, 387-391) and are commercially available, such as a mutant *yca1,* in which the complete ORF is missing, used in the present examples. Alternatively, genomic deletions of specific genes can be made in suitable yeast strains, such as, but not limited to, the *S*. *cerevisiae* W303-1A strain (Thomas and Rothstein (1989) Cell 56, 619-630), the BY4741 strain (Brachmann et al. (1998) Yeast 14, 115-132) or the ∑1278b strain (Kron, (1997) Trends Microbiol. 5, 450-454) by PCR product-directed gene disruption as described previously (Brachmann et al. (1998) Yeast 14, 115-132) using specific oligonucleotides and the pRS vectors as templates for auxotrophic selectable markers. Deletions can then be checked by Southern Blot analysis (Sambrook et al. Molecular Cloning, a Laboratory Manual, 2nd edn. 1989; Cold Spring Harbor, N.Y.: Cold Spring Harbor Laboratory Press) or by PCR analysis.

**Western analysis:** Yeast cell cultures were grown till an OD₆₀₀ of 2-3. Cells were harvested by centrifugation, washed in sterile water and the pellets were resuspended in extraction buffer containing 10 mM Tris-HCl, pH 8.0, 150mM NaCl, 0.05% Tween 20, 10% Glycerol, 5mM EDTA, 1mM DTT and a mixture of protease inhibitors (Complete, Roche Molecular Biochemicals, Germany) and disrupted by vortexing for 5 minutes in the presence of glass beads. The resulting suspension was spun down in a microfuge at maximum speed and part of the resulting supernatant was taken up in loading buffer, fractionated by a 15% SDS-PAGE (7) and blotted onto PVDF membrane.

**Immunodetection of proteins** was carried out using mouse anti-alpha-synuclein monoclonal antibody (Zymed Laboratories Inc., USA) and donkey anti-mouse IgG polyclonal antibody conjugated with alkaline phosphatase (Rockland, USA). Proteins were visualized using NBT/BCIP ready-to-use tablets (Roche) according to the manufacturer's instructions.

**Growth assay:** Spotting experiments: yeast cells were routinely grown overnight at 30°C at room temperature in selective media until they reached the log or late log phase. Cell density was determined using a spectrofotometer and diluted to an OD₆₀₀ of 6. Ten-fold serial dilutions were spotted onto solid media and incubated for 48 hours at 30°C.

### EXAMPLE 1 - EXPRESSION OF GENES ENCODING HUMAN ALPHA-SYNUCLEIN AND MUTANT DERIVATIVES THEREOF IN YEAST: FUNCTIONAL YEAST EXPRESSION PLASMIDS FOR ALPHA-SYNUCLEIN

To demonstrate that the genes alpha-synuclein, alpha-synuclein(A53T) and alpha-synuclein(A30P) can be expressed in yeast Western analysis was performed on yeast extracts of cells transformed with alpha-synuclein expression vectors to visualize the corresponding proteins. For detection a monoclonal anti-alpha-synuclein was used.

Results: As shown in Figure 1 a successful expression of alpha-synuclein and its mutant derivatives was obtained in yeast. The expression levels were found similar between the alpha-synuclein derivatives.

### EXAMPLE 2 - EXPRESSION OF GENES ENCODING HUMAN ALPHA-SYNUCLEIN AND MUTANT DERIVATIVES THEREOF IN YEAST INHIBITS GROWTH: IRON OR ZINC-DEPENDENT CYTOTOXICITY OF ALPHA-SYNUCLEIN.

The influence of metal ions on the effect of alpha-synuclein expression in the cell was investigated. To this end, the growth of yeast cells expressing genes encoding alpha-synuclein and mutant versions thereof in the presence of zinc ions and iron ions in the growth medium was analysed.

Results: Yeast cells producing alpha-synuclein displayed a strongly reduced growth on solid growth medium with 16 mM zinc ions (Fig. 2A). This effect is specifically elicited by alpha-synuclein since growth of the control strains was found unaffected significantly by zinc ions. In addition growth on liquid media was tested (Fig. 2B). Cells producing alpha-synuclein were growing slower or were even completely inhibited for growth in medium supplemented with zinc ions or iron relative to the control strain which does not express alpha-synuclein cDNA. Cells producing alpha-synuclein(A30P) appeared to be slightly less sensitive for the presence of zinc ions in the medium. Apart from that, no major differences are observed between strains producing wild type synuclein and the mutant derivatives.

### EXAMPLE 3 - EXPRESSION OF THE GENE ENCODING HUMAN ALPHA-SYNUCLEIN IN A YEAST STRAIN DELETED FOR YCA1: ALPHA-SYNUCLEIN HYPERSENSITIVITY IN YCA1 CELLS.

To study the effect of yeast caspase Yca1 on alpha-synuclein properties in yeast, the gene encoding alpha-synuclein was expressed in *yca1* mutants and growth on iron ion-containing growth medium was determined (Fig 3).

Results: *yca1* cells producing alpha-synuclein ("*yca1*(aSYN)") grew significantly slower on both solid and liquid medium without iron ions relative to the corresponding wild type strain producing alpha-synuclein (wild type (aSYN)"). Also on medium containing iron ions, growth of *yca1* cells expressing alpha-synuclein is more strongly inhibited than that of the corresponding wild type strain. This Yca1-dependent effect was found to require the presence of intracellular alpha-synuclein specifically, since *yca1* cells transformed with the control vector ("*yca1*(212T)") displayed a similar growth rate, or even a slightly better growth rate than the corresponding wild type yeast strains ("wild type (212T)"). Thus the absence of Yca1 activity renders yeast cells hypersensitive for intracellular produced alpha-synuclein.

### EXAMPLE 4 - ASSESSMENT OF AN YEAST-BASED MODEL OF AMYLOIDOSES IN HIGH-THROUGHPUT SCREENS: Selection of compounds that suppress α-synuclein toxicity in yeast.

The usability of the disclosed yeast model was examined in high-throughput screens. To this end, 500 compounds were tested individually to address their capacity to modulate α-synuclein toxicity in *yca1* yeast cells. Yeast cells were grown in 96-well microtiter plates and growth was monitored by measuring OD at 600nm. The growth medium contained 2mM FeCl₃. The compounds were dissolved in DMSO and added to the growth medium at a final concentration of 10µg/ml. Every microtiter plate contained positive and negative controls for calculating the Z'-factor in order to assess the quality of the assay of each microtiter plate. Positive and negative growth controls were *yca1* cells transformed with 212T(V) and 212T-aSYN(WT), respectively. 500 compounds were screened in duplicate (screen 1 and 2). The OD₆₀₀ values were normalised to the negative control and thus represent the growth relative to yca *1* cells that produce α-synuclein.

The assay was done in duplicate and the effect of each compound on growth was determined by measuring optical density at 600 nm (OD₆₀₀). The OD₆₀₀ measurements were normalised and depicted as a scatter graph (Fig. 4). The Z'-factor (Zhang et al. (1999) J. Biomol. Screen. 4, 67-73) was calculated for each microtiter plate in order to obtain an quantitative score of the assay quality.

Results: The average Z'-factor of each plate was found at least 0,70 or higher indicating that the assay is robust and reproducible and therefore very suitable for high-throughput screening purposes. Consistently, testing 500 compounds resulted in highly reproducible OD₆₀₀ values for each compound (Fig 4). Importantly, this assay allowed selection of compounds that significantly and reproducibly improved growth of the yeast strain.

## Claims

1. An engineered yeast cell, comprising a transgene encoding one or more amyloidogenic proteins or mutant(s) thereof, wherein said yeast strain is **characterized in that** it lacks a functional caspase Yca1 gene.

2. The engineered yeast cell according to claim 1, wherein the one or more amyloidogenic proteins or mutant(s) thereof are encoded by a minigene.

3. The engineered yeast cell according to claim 1 or 2, wherein said amyloidogenic protein is alpha-synuclein.

4. Use of the engineered yeast cell according to any of claims 1 to 3, as a model for amyloidoses.

5. The use according to claim 4, wherein the model for amyloidoses is a model for Parkinson's disease or Lewy body disease.

6. Use of the engineered yeast cell according to any of claims 1 to 3, for screening compounds capable of affecting the toxicity of amyloidogenic proteins in yeast.

7. A method for identifying a compound which influences the toxic effect of amyloidogenic proteins in yeast, said method comprising the steps of:
a) providing an engineered yeast strain, comprising a transgene or a minigene encoding one or more amyloidogenic proteins or mutant(s) thereof, wherein said yeast strain is **characterized in that** it lacks a functional Yca1 gene,
b) contacting the yeast strain obtained in step (a) with said compound, and
c) determining the phenotypic effect of said compound on said yeast.

8. The method according to claim 7 wherein step b) is performed in a medium comprising metal ions.

9. The method according to claim 7 or 8, wherein said amyloidogenic protein is alpha-synuclein.

## Patentansprüche

1. Gentechnisch veränderte Hefezelle, umfassend ein Transgen, das für eines oder mehrere amyloidogene Proteine oder (eine) Mutante(n) davon kodiert, wobei der Hefestamm **dadurch gekennzeichnet ist, dass** ihm ein funktionales Caspase-Ycal-Gen fehlt.

2. Gentechnisch veränderte Hefezelle nach Anspruch 1, wobei das eine oder die mehreren amyloidogenen Proteine oder (die) Mutante(n) davon von einem Minigen kodiert werden.

3. Gentechnisch veränderte Hefezelle nach Anspruch 1 oder 2, wobei das amyloidogene Protein alpha-Synuclein ist.

4. Verwendung der gentechnisch veränderten Hefezelle gemäß einem der Ansprüche 1 bis 3 als Modell für Amyloidosen.

5. Verwendung nach Anspruch 4, wobei das Modell für Amyloidosen ein Modell für Parkinson-Krankheit oder Lewy-Körperchen-Krankheit ist.

6. Verwendung der gentechnisch veränderten Hefezelle gemäß einem der Ansprüche 1 bis 3 zum Screenen nach Verbindungen, die dazu in der Lage sind, die Toxizität von amyloidogenen Proteinen in Hefe zu beeinflussen.

7. Verfahren zum Identifizieren einer Verbindung, die den toxischen Effekt von amyloidogenen Proteinen in Hefe beeinflusst, wobei das Verfahren die Schritte umfasst:
a) Bereitstellen eines gentechnisch veränderten Hefestamms, der ein Transgen oder ein Minigen umfasst, das für eines oder mehrere amyloidogene Proteine oder (eine) Mutante(n) davon kodiert, wobei der Hefestamm **dadurch gekennzeichnet ist, dass** ihm ein funktionales Ycal-Gen fehlt,
b) In-Kontakt-Bringen des in Schritt (a) erhaltenen Hefestamms mit der Verbindung und
c) Bestimmen des phänotypischen Effekts der Verbindung auf die Hefe.

8. Verfahren nach Anspruch 7, wobei der Schritt b) in einem Medium durchgeführt wird, das Metallionen enthält.

9. Verfahren nach Anspruch 7 oder 8, wobei das amyloidogene Protein alpha-Synuclein ist.

## Revendications

1. Cellule de levure manipulée, comprenant un transgène codant pour une ou plusieurs protéines amyloïdogènes ou un ou des mutant(s) de celle(s)-ci, dans laquelle ladite souche de levure est **caractérisée en ce qu'**elle est dépourvue d'un gène de caspase Yca1 fonctionnel.

2. Cellule de levure manipulée selon la revendication 1, dans laquelle la ou plusieurs protéines amyloïdogènes ou le(les) mutant(s) de celle(s)-ci sont codé(e)s par un minigène.

3. Cellule de levure manipulée selon la revendication 1 ou 2, dans laquelle ladite protéine amyloïdogène est l'alpha-synucléine.

4. Utilisation de la cellule de levure manipulée selon l'une quelconque des revendications 1 à 3, en tant que modèle pour des amyloïdoses.

5. Utilisation selon la revendication 4, dans laquelle le modèle pour des amyloïdoses est un modèle pour la maladie de Parkinson ou pour la maladie à corps de Lewy.

6. Utilisation de la cellule de levure manipulée selon l'une quelconque des revendications 1 à 3, pour le criblage de composés susceptibles d'influer sur la toxicité de protéines amyloïdogènes dans la levure.

7. Procédé d'identification d'un composé qui influence l'effet toxique de protéines amyloïdogènes dans la levure, ledit procédé comprenant les étapes de :
a) fourniture d'une souche de levure manipulée, comprenant un transgène ou un minigène codant pour une ou plusieurs protéines amyloïdogènes ou un ou des mutant(s) de celle(s)-ci, dans lequel ladite souche de levure est **caractérisée en ce qu'**elle est dépourvue d'un gène Yca1 fonctionnel,
b) mise en contact de la souche de levure obtenue dans l'étape (a) avec ledit composé, et
c) détermination de l'effet phénotypique dudit composé sur ladite levure.

8. Procédé selon la revendication 7 dans lequel l'étape b) est effectuée dans un milieu comprenant des ions métalliques.

9. Procédé selon la revendication 7 ou 8, dans lequel ladite protéine amyloïdogène est l'alpha-synucléine.
